(19) **Europäisches Patentamt / European Patent Office / Office européen des brevets**

(11) **EP 3 082 594 B1**

(12) # EUROPEAN PATENT SPECIFICATION

(45) Date of publication and mention
of the grant of the patent:
**05.01.2022 Bulletin 2022/01**

(21) Application number: **13824421.5**

(22) Date of filing: **20.12.2013**

(51) Int Cl.:
***A61B 5/083*** (2006.01)

(86) International application number:
**PCT/SE2013/051587**

(87) International publication number:
**WO 2015/094060 (25.06.2015 Gazette 2015/25)**

(54) **METHOD AND APPARATUS FOR ESTIMATING SHUNT**

VERFAHREN UND VORRICHTUNG ZUR SCHÄTZUNG EINES SHUNTS

PROCÉDÉ ET APPAREIL POUR ESTIMER UN SHUNT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**26.10.2016 Bulletin 2016/43**

(73) Proprietor: **Maquet Critical Care AB
171 06 Solna (SE)**

(72) Inventors:
  • **SUAREZ SIPMANN, Fernando
    28010 Madrid (ES)**
  • **TUSMAN, Gerardo
    7600 Mar del Plata (AR)**
  • **SANTOS OVIEDO, Arnoldo
    28003 Madrid (ES)**
  • **BÖHM, Stephan H.
    21481 Lauenburg (DE)**

(74) Representative: **Zacco Sweden AB
Valhallavägen 117
Box 5581
114 85 Stockholm (SE)**

(56) References cited:
**US-A- 6 042 550**

  • **CAPEK J M ET AL: "NONINVASIVE
    MEASUREMENT OF CARDIAC OUTPUT USING
    PARTIAL CO2 REBREATHING", IEEE
    TRANSACTIONS ON BIOMEDICAL
    ENGINEERING, IEEE SERVICE CENTER,
    PISCATAWAY, NJ, USA, vol. 35, no. 9, 1
    September 1988 (1988-09-01), pages 653-661,
    XP000209300, ISSN: 0018-9294, DOI:
    10.1109/10.7266**
  • **"Volumetric Capnography - The Next Advance in
    CO2 Monitoring", , 31 January 2012 (2012-01-31),
    XP055108966, Retrieved from the Internet:
    URL:http://oem.respironics.com/wp/Volumetr ic
    Capnography.pdf [retrieved on 2014-03-19]**

**Description**

TECHNICAL FIELD

The present invention relates to a method, an apparatus and a computer program for estimating shunt, and in particular to a method, apparatus and computer program for minimally invasive estimation of shunt based on carbon dioxide measurements.

BACKGROUND

Human cells need oxygen ($O_2$) to live because they obtain energy by consuming $O_2$ and glucose throughout aerobic metabolism. The lungs take $O_2$ molecules from air during breathing, which diffuse into capillary blood through the alveolar-capillary membrane - a passive process called gas exchange. $O_2$ molecules then bind to haemoglobin and are transported by the blood assuring an optimal $O_2$ delivery to all body cells.

Gas exchange at the lung level is the key process and it depends on the close matching of ventilation delivering $O_2$ to the gas exchange surface, the alveolar-capillary membrane, and blood perfusion taking up oxygen and offloading carbon dioxide. Ventilation-perfusion (V/Q) mismatch is the underlying cause of most gas exchange abnormalities and is often a result of pulmonary and cardiovascular diseases.

In this context, *shunt* is an important physiological parameter. There are numerous of different and often inconsistent definitions of shunt in the medical literature. In this application, shunt is the sum of anatomic shunt and pulmonary shunt. *Anatomic shunt* is the fraction of blood bypassing the alveoli of the lungs through anatomic channels. The anatomic shunt is often referred to as normal shunt or physiological shunt and is related to the anatomical fact that the blood of the bronchial veins and the Thebesian veins drain in the left heart without undergoing gas change in the pulmonary capillaries. The anatomic shunt accounts for approximately 2% to 4% of the normal cardiac output. *Pulmonary shunt* is the fraction of pulmonary blood flow perfusing the alveoli of the lungs but not participating in gas exchange due to insufficient ventilation, i.e. the fraction of total shunt caused by zero or low V/Q ratio. Thus, in this application, pulmonary shunt corresponds to what is often referred to as *venous admixture,* which includes blood passing through both zero V/Q areas and low (non-zero) V/Q areas of the lung. *Pure pulmonary shunt* (or simply pure shunt) is the part of cardiac output passing through zero V/Q areas of the lung, i.e. areas where V/Q = 0. Here it should be noted that in some medical literature the term shunt only encompasses pure shunt (i.e. zero V/Q) and not blood from V/Q heterogeneity areas (i.e. low V/Q areas). V/Q mismatch is caused either by pulmonary shunt (low or zero V/Q) or dead space (high or infinite V/Q).

The result of pulmonary shunt is an impaired blood oxygenation known as hypoxemia (i.e. a decrease in $O_2$ content in arterial blood), caused by the shunted venous blood (with low $O_2$ content) that reaches the systemic arterial side without contacting the ventilated alveoli rich in $O_2$. This poorly oxygenated blood decreases the amount of $O_2$ delivered to body cells and can affect the normal aerobic metabolism.

Taking into account the above explanations, the measurement of shunt is considered the gold standard for assessing blood oxygenation in critical care medicine. It integrates information regarding lung ventilation and perfusion and allows the assessment of the lung's efficiency in oxygenating blood. This index is a useful parameter that helps clinicians understand the primary cause of gas exchange abnormalities, to make differential diagnosis and to guide treatment in their patients. Therefore, the calculation of shunt is essential to assess pulmonary function in critically ill patients undergoing mechanical ventilation and has been related to their outcome.

The reference method to measure shunt in clinical practice is based on the measurement of arterial and mixed venous oxygen contents by means of the pulmonary artery catheter (PAC). Taking simultaneous arterial and mixed-venous blood samples shunt can be calculated by Berggren's equation[1], sometimes referred to as the pulmonary shunt equation, as:

$$\text{shunt (\%)} = \frac{CcO_2 - CaO_2}{CcO_2 - C\overline{v}O_2} \qquad\qquad \text{(Eq.1)}$$

where $CcO_2$, $CaO_2$ and $C\overline{v}O_2$ are the contents of $O_2$ in pulmonary capillaries, arterial and mixed venous blood, respectively.

However, the above described method and equation for shunt determination has a number of shortcomings:

1) It is an invasive monitoring that is rarely justified even in most critically ill patients. This is because PAC is associated with potential severe complications like sepsis, pulmonary infarction, bleeding and arrhythmias among others. Besides, the use of PAC has significantly declined because its use has repeatedly failed to improve the outcome of critically ill patients.

2) The oxygen content method cannot measure $CcO_2$ directly. This value is calculated based on the assumption that capillary blood is fully saturated. However, this assumption might not be true even if 100% inspired oxygen fraction ($FiO_2$) were used.

3) When using a $FiQ_2 < 1$, this method becomes only a rough estimate of venous admixture.

There are also other methods for measuring shunt, such as the Multiple Inert Gas Elimination Technique and methods which create ventilation-perfusion maps by imaging and nuclear medicine methodologies. However, these methods are cumbersome, costly, time-consuming and impossible to apply at the bedside and, therefore, they cannot be considered clinical monitoring methods.

Due to said shortcomings, the above mentioned methods fail to easily and reliably provide an indication of shunt in mechanically ventilated patients in operating theatres or in intensive care units. Therefore, several indexes that are more easily obtainable at the bedside, like the $PaO_2$-$FiO_2$ ratio, the alveolar to arterial gradient of $PO_2$ ($AaPO_2$) and the respiratory index, have been introduced in daily practice as a surrogate of shunt at the bedside. Despite being widely used, most physicians agree that these indexes are not real substitutes of shunt in critically ill patients undergoing complex clinical processes.

Also other basic and less reliable approximations in the estimation of shunt have been discussed, e.g. in publications 2 to 6 in the list of references appended hereinafter. The estimations discussed in these publications are gross calculations based on simplistic and, many times unrealistic assumptions and, therefore, their clinical use is questionable.

There are numerous studies relating to estimation of physiological parameters playing an important role in pulmonary gas exchange, several of which are relevant to the present invention. For example, Suarez-Sipmann et al.[7] shows that the so called Bohr dead space (sometimes referred to as "true dead space") can be reliably estimated using Bohr's formula when $PACO_2$ is determined through volumetric capnography, and that Enghoff's modification of Bohr's formula (the Bohr-Enghoff's formula) using the concept of ideal $PACO_2$ ($PACO_2 = PaCO_2$) tends to overestimate dead space due to inclusion of the shunt effect.

There is also patent literature related to the estimation of physiological parameters that are relevant to the present invention. For example, WO 2012/069051 discloses a device for determining two or more respiratory parameters relating to an individual, e.g. an individual suffering from pulmonary gas exchange problems. The device has detection means for oxygen and carbon dioxide contents in inspired and expired gas and blood. The device is controlled by a computer with functionality for entering oxygenation, carbon dioxide and acid-base values from one or more blood samples from arterial, venous, central venous or mixed venous blood samples, and with the parameter estimation based on equations of gas exchange of both oxygen and carbon dioxide and equations describing the acid-base chemistry of blood potentially including the competitive binding of oxygen and carbon dioxide to haemoglobin.

Furthermore, minimally invasive oxygen based approaches for calculating shunt have been described in e.g. US 6042550, and Peyton et al[8]. However, calculating shunt from $O_2$-related parameters has been proved difficult and uncertain since this requires several assumptions to be made regarding unknown physiological parameters, as will be discussed in more detail in the specification following hereinafter.

SUMMARY OF THE INVENTION

It is an object of the present invention to provide means for a reliable estimation of the shunt of a subject, and in particular the pulmonary shunt of the subject.

It is another object of the invention to provide such means that eliminates or at least mitigates one or more of the shortcomings associated with prior art described above.

It is yet another object of the invention to provide means for enabling reliable monitoring of shunt in clinical practice, e.g. for enabling monitoring of shunt of patients undergoing ventilatory treatments. It is a particular object of the invention to provide means for a reliable estimation of the shunt of a subject, through which means it is possible to estimate the shunt of the subject in a way that is minimally invasive.

The scope of protection of the present invention is defined by the subject-matter of claims 1 to 15.

These and other objects are achieved by means of a method for estimating shunt of a subject, such as a human subject undergoing ventilatory treatment. The method involves determination of shunt at least partly based on a measured carbon dioxide ($CO_2$) in the expiration gas exhaled by said subject. The method comprises the steps of:

- obtaining, from $CO_2$ measurements on expiration gas exhaled by said subject, a first value related to alveolar $CO_2$ of said subject;
- obtaining a second value related to arterial $CO_2$ of said subject;
- obtaining a third value related to cardiac output ($Q_T$) or effective pulmonary perfusion (EPP) of said subject;
- obtaining a fourth value related to $CO_2$ elimination ($VCO_2$) of said subject, and

- calculating the shunt of the subject based on said first, second, third and fourth values.

The objects are also achieved by an apparatus devised and configured to carry out the method, and a computer program for causing the apparatus to carry out the method when executed by a processing unit of the apparatus.

The invention makes use of the fact that a value related to $Q_T$ or EPP, the latter sometimes also referred to as effective pulmonary blood flow (EPBF) or pulmonary capillary blood flow ($Q_{pcbf}$), may be introduced and used in the calculation of shunt to eliminate the need for invasive venous blood samples, required in most methods for determination of shunt according to prior art.

The calculation of shunt is preferably based on a combination of the Fick principle for calculation of cardiac output or effective pulmonary perfusion and a modification of Berggren's equation for calculation of shunt where in the formula $O_2$ is replaced by $CO_2$ and the equation is rearranged accordingly. The Fick principle for cardiac output (Eq. 2A), the Fick principle for effective pulmonary perfusion (Eq. 2B) and the modified Berggren equation for $CO_2$ based calculation of shunt (Eq. 3) are shown below.

$$Q_T \left( CvCO_2 - CaCO_2 \right) = VCO_2 \qquad\qquad (Eq.\ 2A)$$

$$EPP \left( CvCO_2 - CcCO_2 \right) = VCO_2 \qquad\qquad (Eq.\ 2B)$$

where $Q_T$ is the cardiac output, EPP is the effective pulmonary perfusion, $CvCO_2$ is the venous $CO_2$ content, $CcCO_2$ is the capillary $CO_2$ content and $VCO_2$ is the $CO_2$ elimination.

$$shunt\ (\%) = \frac{CaCO_2 - CcCO_2}{CvCO_2 - CcCO_2} \qquad\qquad (Eq.3)$$

where $CaCO_2$ is the arterial $CO_2$ content, $CcCO_2$ is the capillary $CO_2$ content and $CvO_2$ is the venous $CO_2$ content.

By combining equations 2A or 2B with equation 3, the denominator ($CvCO_2 - CcCO_2$) in equation 3 can be eliminated, allowing shunt to be estimated without invasive procedures for obtaining values of $CvCO_2$, thereby enabling shunt to be calculated and monitored in a minimally invasive way.

Another important aspect of the invention is the conversion of measurable alveolar partial pressure, concentration or volume of $CO_2$ into capillary $CO_2$ content of the subject. Preferably, the first value related to alveolar $CO_2$ is a value of alveolar $CO_2$ partial pressure, concentration or volume substantially corresponding to a capillary $CO_2$ partial pressure, concentration or volume of the subject. Such a value may be directly obtained from the $CO_2$ measurements on the expiration gas exhaled by the subject, e.g. by means of volumetric capnography allowing a value of alveolar partial pressure of $CO_2$ ($PACO_2$) which corresponds to a capillary partial pressure of $CO_2$ ($PcCO_2$) to be determined from a volumetric capnogram derivable from the volumetric capnography. The alveolar $CO_2$ partial pressure, concentration or volume substantially corresponding to a capillary $CO_2$ partial pressure, concentration or volume of the subject may then be used to estimate capillary $CO_2$ content using a known value of $CO_2$ solubility in blood. In this way, the $CcCO_2$ value in the numerator of the $CO_2$-based Berggren equation (Eq. 3) can be replaced by said first value related to alveolar $CO_2$ and known parameters relating $CO_2$ content to $CO_2$ partial pressure. More exactly, as will be described in the detailed description following hereinafter, the inventive concept involves the introduction of the term $S(PaCO_2 - PACO_2)$, where $S$ is the solubility of $CO_2$ in blood, $PaCO_2$ the arterial partial pressure of $CO_2$ and $PACO_2$ the alveolar partial pressure of $CO_2$, as representative for the arterial to capillary content difference used in the numerator of the $CO_2$-based Berggren equation (Eq. 3), which allows shunt to be determined without determination of the subject's capillary $CO_2$ content.

An advantage of the proposed method for shunt estimation is that the calculation of shunt can be frequently repeated and carried out at the bedside, allowing reliable monitoring of shunt in clinical practice, e.g. in monitoring shunt of mechanically ventilated patients in operating theatres or in intensive care units.

Another advantage is that the shunt value can be calculated without having to use shunt-related surrogates or rough approximations of physiological parameters that play an important role in the pulmonary gas exchange. Thereby, the shunt value calculated in accordance with the principles of the present invention is believed to better represent the true shunt of the subject than shunt or shunt related parameters clinically available today.

Yet another advantage is that the shunt value can be calculated according to the principles of the present invention without the need for (total or partial) rebreathing. This allows shunt to be calculated independently of the type of therapy currently provided to the patient or the type of breathing circuit or ventilator currently used to provide breathing support to the patient.

Another advantage is that the calculated shunt value is a reliable measure of venous admixture, including shunt caused by both zero V/Q and low V/Q, i.e. including both pure shunt and V/Q heterogeneity. This provides more robustness in its interpretation both for diagnostic (i.e. classification) and therapeutic purposes. This means that, together with dead space measurements according to known principles, the shunt value calculated according to the principle of the invention provides information on the full spectrum of V/Q abnormalities (i.e. relative contributions of low or high V/Q to a given condition or response to therapeutic intervention).

As arterial $CO_2$ content is used in the calculation, the calculated shunt value is affected by small contributions of the anatomical pathways by which un-oxygenated venous blood containing relatively high amounts of $CO_2$ reaches the arterial (left heart) side. However, since this anatomic shunt constitutes only a very small fraction of total shunt, the calculated shunt value is thus a good measure of the pulmonary shunt fraction of total shunt. If desirable, the calculated shunt value may of course be adjusted by compensating for the contribution of the anatomic shunt. However, since the anatomic shunt typically remains rather constant, continuous monitoring of the shunt value calculated in accordance with the principles of the invention still provides reliable indications of changes in the pulmonary shunt of the subject.

Yet another advantage of the proposed principle for calculating shunt is that it is less sensitive to variations in $FiO_2$ than methods employing Berggren's original $O_2$-based equation (Eq. 1). This is due to the fact that the oxygen content of blood flowing through low V/Q areas is very sensitive to the level of $FiO_2$ used because higher $FiO_2$ increases the $O_2$ diffusion gradient across the alveoli thereby underestimating the true venous admixture. Thus, using a higher $FiO_2$ in low V/Q alveoli can, to a certain extent, compensate for the reduced ventilation and result in similar values for $CcO_2$ and $CaO_2$ so that Berggren's $O_2$ based equation (Eq. 1) cannot "see" these low V/Q areas. By using a $FiO_2$ of 1.0 this compensatory effect is maximized and Berggren's equation almost only measures the zero V/Q or very low V/Q portions of the lung. Furthermore, when using 100% oxygen the poorly ventilated alveoli tend to collapse (as the only gas is oxygen that is rapidly consumed, the so called "reabsorption atelectasis") and these units then become zero V/Q units, further reducing the contribution of low V/Q zones. $CO_2$ always has a high gradient in the opposite direction (as $CO_2$ in the inhaled air is always very low) and is also twenty-two times more soluble than oxygen so it diffuses much better. Therefore, even though the level of oxygenation affects $CO_2$ release from the blood (in fact, the higher the oxygen the more likely the $CO_2$ will abandon the blood and enter the alveoli, something called the Haldane effect) this effect is only very limited, would only depend on the level of oxygenation in the blood and not in the alveoli, and is therefore unlikely to have much influence on the shunt value calculated in accordance with the proposed principles.

The proposed method is a completely $CO_2$ based method for estimating shunt, meaning that the value of shunt is calculated only through analysis of $CO_2$ transport between the lungs and the blood of the subject. The method does not involve analysis of $O_2$ transport. Using a different wording, the shunt value is calculated using nothing but $CO_2$ related parameters, i.e. without using $O_2$ related parameters such as the arterial $O_2$ content ($CaO_2$), the capillary $O_2$ content ($CcO_2$), the alveolar $O_2$ content ($CAO_2$), the oxygen uptake ($VO_2$), the capillary oxygen saturation ($ScO_2$) and the fraction of inspired $O_2$ ($FiO_2$).

Using a $CO_2$-based approach for shunt calculation is advantageous compared to an $O_2$ based approach for several reasons. First, determination of $VCO_2$ (i.e. $CO_2$ elimination), which requires sufficient temporal resolution and synchronization between flow or volume and concentration or partial pressure determination, is less cumbersome than the determination of $VO_2$ (i.e. $O_2$ uptake). It is possible to estimate $VO_2$ from $VCO_2$ but this introduces uncertainties in the shunt calculation since it requires the patient's respiratory quotient (RQ) to be assumed, which quotient typically varies between 0.7-1.0. Secondly, the $CO_2$ based approach does not need to assume certain (typically 100%) $O_2$ saturation in capillary blood ($ScO_2$), an assumption that may be erroneous when using low $FiO_2$ levels or if there are diffusion abnormalities in the lungs of the patient. Thirdly, the $CO_2$ based approach does not require a measurement or any assumption of haemoglobin concentration and haemoglobin capacity values, nor does it require chemical analysis of blood in order to determine such values. Furthermore, the $CO_2$-based approach does not require calculation of alveolar $O_2$ content, which alveolar $O_2$ content, using an $O_2$-based approach, has to be calculated based on e.g. $FiO_2$ and the above mentioned RQ and haemoglobin values. Instead, using the proposed $CO_2$ based approach for calculating shunt, a value related to alveolar $CO_2$ of the subject that can be used to estimate the capillary $CO_2$ content is derivable directly from the $CO_2$ measurements on the expiration gas.

The proposed method involves volumetric capnography, meaning that at least one of the first alveolar $CO_2$ related value, the second arterial $CO_2$ related value, the third $Q_T$ or EPP related value and the fourth $VCO_2$ related value is determined based on data obtained through capnography, and preferably volumetric capnography. Volumetric capnography typically involves measurements of the flow or volume of the expiration gas and the partial pressure, concentration or volume of $CO_2$ in the expiration gas, and calculation of a volumetric capnogram from said measurements.

Preferably, said first value related to alveolar $CO_2$ is a value of $CO_2$ partial pressure ($PACO_2$), concentration or volume, and most preferably a $PACO_2$ value. This value may be determined based on the $CO_2$ measurements on the expiration gas, preferably by means of said volumetric capnography. In a preferred embodiment, said first value is set to a $CO_2$ value found at or near the midpoint of the alveolar slope (phase III) of the volumetric capnogram, which value corresponds to a $PACO_2$ value reflecting the capillary partial pressure of $CO_2$ ($PcCO_2$). As mentioned above, this value related to alveolar $CO_2$ may then be used to eliminate the term $CcCO_2$ from the $CO_2$-based Berggren equation (Eq. 3) in order to calculate shunt without having to determine the capillary $CO_2$ content of the subject.

Preferably, also the fourth value indicative of $CO_2$ elimination is determined based on $CO_2$ measurements on the expiration gas, advantageously through said volumetric capnography. For example, a value of $VCO_2$ may be calculated from a capnogram, preferably a volumetric capnogram, and a value indicative of the respiratory rate (RR) of the patient. In a preferred embodiment, the $CO_2$ elimination is determined as the area under the curve of the capnogram multiplied by the respiratory rate of the subject.

The third value indicative of $Q_T$ or EPP may be obtained by means of any known method for estimating cardiac output or effective pulmonary perfusion. Preferably, the value of $Q_T$ or EPP is non-invasively determined based on the measured $CO_2$ content in the expiration gas. This may be achieved using known capnodynamic methods for $Q_T$ or EPP determination. It is also possible to use an independent method for determining a $Q_T$ or EPP related value, i.e. a method that does not use the measurements of $CO_2$ content in the expiration gas exhaled by the subject in the $Q_T$ or EPP determination, whereby the independently determined value of $Q_T$ or EPP may be input to the apparatus of the present invention by an operator, and used by the apparatus in the calculation of shunt.

Preferably, said second value related to arterial $CO_2$ is a value of arterial $CO_2$ partial pressure ($PaCO_2$), concentration or volume, and most preferably a $PaCO_2$ value. As of today, there are available methods for non-invasively estimating arterial partial pressure of $CO_2$, such as transcutaneous $CO_2$ measurements. However, these methods may not reliably determine $PaCO_2$ under all clinical circumstances. Therefore, this value is preferably obtained through an arterial blood sample, whereby the $PaCO_2$ value derived from the blood sample may be input by an operator to the apparatus carrying out the method in order for the apparatus to use the $PaCO_2$ value in the calculation of shunt. However, in order to make the proposed method completely non-invasive, it is contemplated that known non-invasively obtained surrogates of $PaCO_2$, such as partial pressure of end-tidal $CO_2$ ($PetCO_2$), may be used instead of $PaCO_2$ in the shunt calculation. A value of $PetCO_2$ is directly available from a capnogram, preferably a volumetric capnogram, which makes it suitable for use when the proposed method is implemented as a capnography-based method, and preferably a volumetric capnography-based method, for calculation of shunt. Thus, in some embodiments, the proposed method for shunt calculation may be completely non-invasively performed based only on non-invasive measurements of $CO_2$ content in the expiration gas exhaled by the subject. However, it may be desirable to obtain a $PaCO_2$ value from an arterial blood sample in order to improve the accuracy of the method or for calibration purposes.

In one embodiment, shunt is calculated as:

$$\text{shunt (\%)} = \frac{S\,(PaCO_2\text{-}PACO_2)}{S\,(PaCO_2\text{-}PACO_2) + \dfrac{VCO_2}{Q_T}} \tag{Eq.4}$$

where 5 is the $CO_2$ solubility, $PaCO_2$ is the partial pressure of arterial $CO_2$, $PACO_2$ is the partial pressure of alveolar $CO_2$, $VCO_2$ is the minute elimination of $CO_2$ and $Q_T$ is the cardiac output.

In another embodiment, the value of cardiac output is replaced by a value of EPP, which makes it possible to calculate shunt as:

$$\text{shunt (\%)} = \frac{S * EPP\,(PaCO_2\text{-}PACO_2)}{VCO_2} \tag{Eq. 5}$$

where 5 is the $CO_2$ solubility, EPP is the effective pulmonary perfusion, $PaCO_2$ is the partial pressure of arterial $CO_2$, $PACO_2$ is the partial pressure of alveolar $CO_2$ and $VCO_2$ is the minute elimination of $CO_2$.

The parameters $PaCO_2$, $PACO_2$, $VCO_2$, $Q_T$ and EPP may be obtained in any of the above described ways. The $CO_2$ solubility, $S$, is known and substantially constant within the relevant physiological range, typically but not necessarily 35 to 50 mmHg of $CO_2$.

The method described above is typically computer implemented, meaning that the method is performed by an apparatus through execution of a computer program.

Thus, according to one aspect of the invention, there is provided a computer program for estimating shunt of a subject, such as a human subject undergoing ventilatory treatment. The computer program comprises computer readable programming code which, when executed by a processing unit of an apparatus arranged to obtain $CO_2$ measurements on expiration gas exhaled by said subject, causes the apparatus to:

- obtain, from said $CO_2$ measurements, a first value related to alveolar $CO_2$ of said subject;
- obtain a second value related to arterial $CO_2$ of said subject;

- obtain a third value related to cardiac output ($Q_T$) or effective pulmonary perfusion (EPP) of said subject;
- obtain a fourth value related to $CO_2$ elimination ($VCO_2$) of said subject, and
- calculate the shunt of the subject based on said first, second, third and fourth values.

The computer program may further be configured to cause the apparatus to carry out any of the above described steps and calculations.

According to another aspect of the invention there is provided an apparatus for estimating shunt of a subject, such as a human subject undergoing ventilatory treatment. The apparatus is configured to obtain $CO_2$ measurements on expiration gas exhaled by said subject, typically obtained from a sensor arrangement comprised in or connectable to the apparatus. The apparatus comprises a processing unit configured to:

- obtain, from said $CO_2$ measurements on expiration gas exhaled by said subject, a first value related to alveolar $CO_2$ of said subject;
- obtain a second value related to arterial $CO_2$ of said subject;
- obtain a third value related to cardiac output ($Q_T$) or effective pulmonary perfusion (EPP) of said subject;
- obtain a fourth value related to $CO_2$ elimination ($VCO_2$) of said subject, and
- calculate the shunt of the subject based on said first, second, third and fourth values.

Preferably, the sensor arrangement comprises a $CO_2$ sensor for measuring the partial pressure, concentration or volume of $CO_2$ in the expiration gas, and a flow or volume sensor for measuring the flow or volume of expiration gas. The sensor arrangement may form part of a capnograph, and preferably a capnograph configured for volumetric capnography.

The apparatus may comprise a user interface configured to allow an operator to input the value related to arterial $CO_2$ of the subject, such as a $PaCO_2$ value, to the apparatus via said user interface, whereby the processing unit may be configured to use the input value in the calculation of shunt. Thereby, the apparatus can be configured to use a $PaCO_2$ value obtained through an arterial blood sample in the shunt calculation.

The apparatus may be configured to receive also other values via the user interface, and to use the values in the shunt calculation. For example, the apparatus may, in some embodiments, be configured to receive a $Q_T$ or EPP related value determined through an independent method and input by an operator via the user interface, and to use said $Q_T$ or EPP related value in the shunt calculation.

Advantageously the apparatus comprises a display configured to display information related to the calculated value of shunt, e.g. a current value of shunt of the subject and/or a graph showing changes in shunt over time.

Preferably, the shunt value is calculated repeatedly, e.g. on a breath-by-breath basis, and the displayed information related to shunt may be updated accordingly.

In one embodiment, the apparatus is a ventilator that includes or is connectable to the sensor arrangement and configured to calculate the shunt of a subject connected to the ventilator based at least partly on the measurements obtained by the sensor arrangement.

In another embodiment the apparatus is a stand-alone device that includes or is connectable to the sensor arrangement, configured to calculate shunt of a subject that may or may not be connected to a ventilator. The device may be a conventional computer that calculates the shunt of the subject according to the principles of the present invention, and displays information relating to the calculated shunt value on a display of the computer.

According to an advantageous aspect of the invention there is provided an apparatus for estimating the shunt of a subject based on capnography, preferably volumetric capnography. To this end the apparatus comprises or is connectable to a capnograph that measures the flow or volume of expiration gas exhaled by a subject and the partial pressure, concentration or volume of $CO_2$ in the expiration gas. The apparatus may be configured to:

- Determine a first value related to alveolar $CO_2$ of the subject. This value is a value of alveolar $CO_2$ partial pressure, concentration or volume substantially corresponding to a capillary $CO_2$ partial pressure, concentration or volume of the subject. Preferably, the value is determined by the apparatus based on capnographic data obtained by the capnograph, and preferably determined as a $PACO_2$ value corresponding to the $CO_2$ value found at or near the midpoint of the alveolar slope (phase III) of a volumetric capnogram derivable from said capnographic data.
- Determine a second value related to arterial $CO_2$ of the subject, typically determined by the apparatus to correspond to a $PaCO_2$ value obtained through an arterial blood sample and received by the apparatus via a user interface of the apparatus, input via said user interface by an operator of the apparatus, or determined non-invasively by the apparatus based on the $CO_2$ and flow or volume measurements obtained by the capnograph or another sensor arrangement comprised in or connected to the apparatus.
- Determine a third value related to $Q_T$ or EPP of the subject, typically determined by the apparatus non-invasively using a capnodynamic equation or a set of capnodynamic equations and the $CO_2$ and flow or volume measurements obtained by the capnograph or another sensor arrangement comprised in or connected to the apparatus.

- Determine a fourth value related to $CO_2$ elimination ($VCO_2$) of the subject, typically non-invasively determined by the apparatus based on the $CO_2$ and flow or volume measurements obtained by the capnograph or another sensor arrangement comprised in or connected to the apparatus.

- Calculate an actual value of shunt of the subject using the first, second, third and fourth value, and typically also a known value of $CO_2$ solubility in blood.

Further advantageous aspects of the present invention will be described in the detailed description following hereinafter.

BRIEF DESCRIPTION OF THE DRAWINGS

The present invention will become more fully understood from the detailed description provided hereinafter and the accompanying drawings which are given by way of illustration only, and in which:

Fig. 1A illustrates an apparatus for estimating shunt according to an exemplary embodiment of the invention;

Fig. 1B illustrates an apparatus for estimating shunt according to another exemplary embodiment of the invention;

Fig. 2 illustrates schematically a model of pulmonary gas exchange taking place in the alveoli A of the lungs of a subject;

Fig. 3A illustrates the simplified Riley's three-compartment model of the lungs, representing the lungs as three distinguished functional units A-B-C;

Fig. 3B illustrates the conceptual representation of the three functional units A-B-C in Fig. 3A in a volumetric capnogram;

Fig. 3C illustrates a volumetric capnogram and its relation to dead space and shunt effect, and

Fig. 4 is a flow chart illustrating a method for estimating shunt according to the principles of the present invention.

DETAILED DESCRIPTION OF EMBODIMENTS

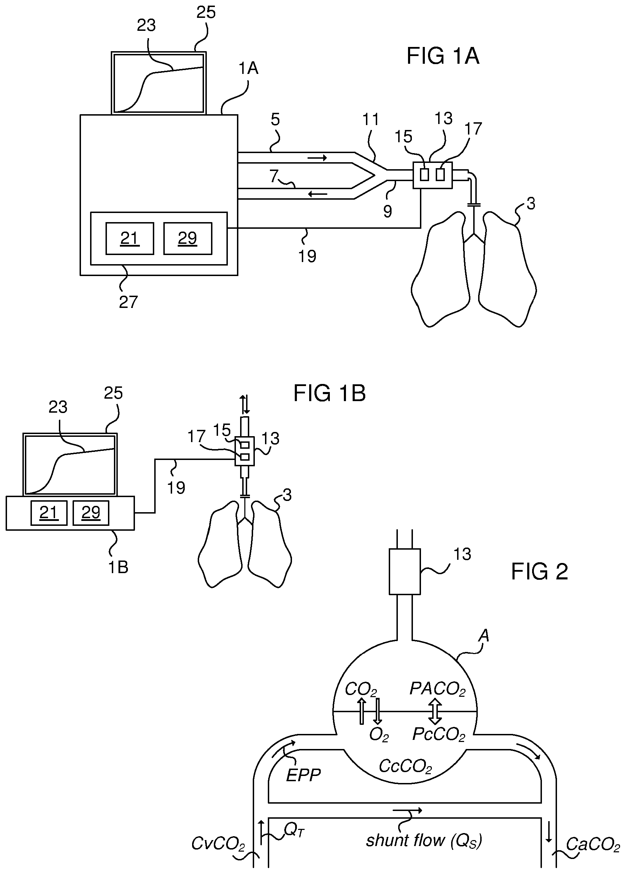

Fig. 1A illustrates an apparatus 1A according to an exemplary embodiment of the invention. In this embodiment the apparatus is a ventilator for providing ventilatory treatment to a patient 3 connected to the ventilator. The ventilator is connected to the patient 3 via an inspiratory line 5 for supplying breathing gas to the patient 3, and an expiratory line 7 for conveying expiration gas away from the patient 3. The inspiratory line 5 and the expiratory line 7 are connected to a common line 9, via a so called Y-piece 11, which common line is connected to the patient 3 via a patient connector, such as an endotracheal tube.

A capnograph 13 configured for volumetric capnography measurements is arranged in the proximity of the airways opening of the patient 3. In this exemplary embodiment, the capnograph 13 is arranged in the common line 9 and exposed to all gas expired and inspired by the patient 3. The capnograph 13 comprises a flow or volume sensor 15 for measuring at least the flow or volume of expiration gas exhaled by the patient 3, and a $CO_2$ sensor 17 for measuring the $CO_2$ content in at least the said expiration gas. Typically but not necessarily the capnograph 13 also measures the flow or volume of inspiration gas inhaled by the patient 3, and the $CO_2$ content in the inspiration gas.

The capnograph 13 is connected to the ventilator via a wired or wireless connection 19, and configured to transmit the flow and $CO_2$ measurements to the ventilator for further processing by a processing unit 21 of the ventilator. The ventilator is preferably configured to generate a volumetric capnogram 23, hereinafter referred to as VCap, from the flow and $CO_2$ measurements received from the capnograph 13, and to display the VCap 23 on a display 25 of the ventilator.

The processing unit 21 is typically part of a control unit 27 of the ventilator, which control unit 27 further comprises a non-volatile memory or data carrier 29 storing a computer program that causes the processing unit 21 to calculate the shunt of the patient 3 in accordance with the principles of the present invention, at least partly based on the flow or volume and $CO_2$ measurements received from the capnograph 13, as will be described in more detail below. The ventilator is further configured to display information related to the calculated shunt value on the display 25. Preferably, the ventilator is configured to repetitively calculate the shunt value, e.g. on a breath-by-breath basis, and to display information on the display 25 enabling a ventilator operator to monitor changes in the shunt of the patient 3.

Fig. 1B illustrates an apparatus 1B according to another exemplary embodiment of the invention. In this embodiment, the apparatus is a conventional computer, connected to the capnograph 13 via the wired or wireless connection 19. Just like the control unit 27 of the ventilator in Fig. 1A, the computer comprises a processing unit 21 and a non-volatile memory or data carrier 29 storing a computer program that causes the processing unit 21 to calculate the shunt of the patient 3 in accordance with the principles of the present invention. In this embodiment, the patient 3 may or may not be connected to a ventilator. The computer also comprises a display 25 for display of VCap and information related to calculated shunt values.

Each of the ventilator 1A and the computer 1B further comprises a user interface 31 through which an operator can enter values of physiological parameters that may be used by the apparatus in the calculation of shunt. For example, a value indicative of arterial $CO_2$ content of the patient 3, such as a $PaCO_2$ value determined from an arterial blood sample, may be input to the apparatus via the user interface 31 and used in the calculation of shunt. Furthermore, in the same way a value indicative of QT or EPP of the patient 3, may be input by the user to the apparatus 1A, 1B via the user interface 31 and used in the calculation of shunt.

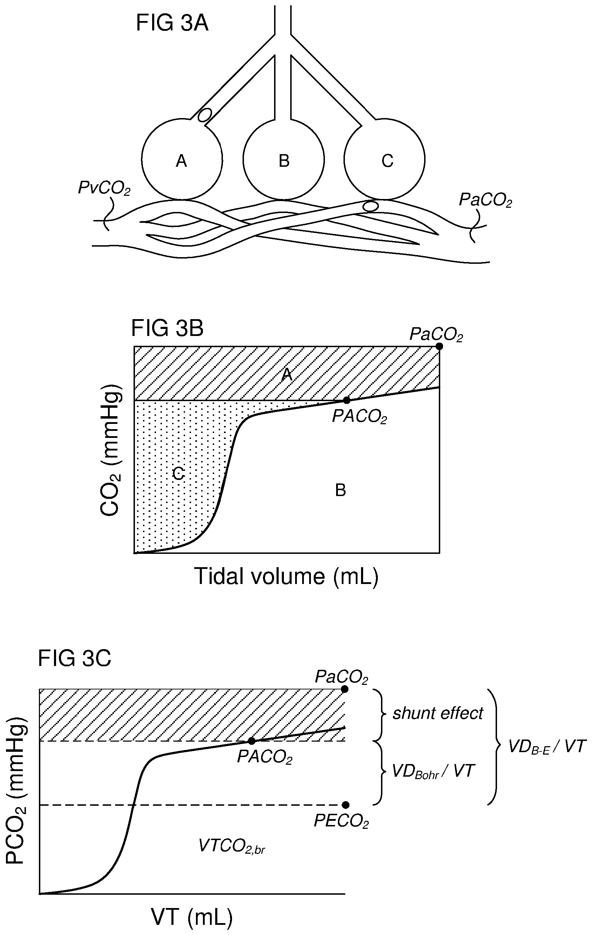

Reference will now be made to Fig. 2 and Figs. 3A-3C, depicting the rationale of the proposed calculation of shunt using volumetric capnography.

Fig. 2 illustrates schematically a model of pulmonary gas exchange taking place in the alveoli A of the lungs of a subject. Venous blood coming from the systemic venous circulation carrying $CO_2$ from the body into the right heart having $CO_2$ content $CvCO_2$ is transported towards the alveoli A in an arterial part of the pulmonary circulatory system. In a capillary part of the pulmonary circulatory system, $CO_2$ moves from the pulmonary capillaries into the alveoli, resulting in $CO_2$ offloading of capillary blood having high $CO_2$ content $CcCO_2$. For most conditions it is reasonable to assume full equilibration between the alveolar partial pressure of $CO_2$ ($PACO_2$) and the capillary partial pressure of $CO_2$ ($PcCO_2$).

Some of the cardiac output ($Q_T$) of the subject does not participate in the gas exchange. The fraction of cardiac output participating in the gas exchange is the effective pulmonary perfusion (EPP), sometimes referred to as the effective pulmonary blood flow (EPBF) or pulmonary capillary blood flow ($Q_{pcbf}$). The fraction of cardiac output that does not participate in the gas exchange is the shunt. The $CO_2$ rich shunt flow (Qs) is mixed with the capillary blood flow from which $CO_2$ was removed to form arterial blood having $CO_2$ content $CaCO_2$, which arterial blood is then transported to a venous part of the pulmonary circulatory system to the left heart and pumped into the systemic arterial circulation and into the organs of the subject.

Fig. 3A shows the simplified Riley's three-compartment model of the lungs[9], which represents the lungs as three distinct functional units: A) a shunt unit with perfusion but without ventilation, i.e. with zero V/Q, B) a normal unit which is normally ventilated and perfused, and C) a dead space high V/Q unit with ventilation but without perfusion, i.e. where V/Q approaches infinity.

Fig. 3B illustrates the representation of the three functional units A, B and C in the volumetric capnogram. The area under the curve of the volumetric capnogram is originated by the normally ventilated and perfused areas of the lungs (unit B) because this part of the lung is the one that receives $CO_2$ from pulmonary capillaries and efficiently eliminates the $CO_2$ by ventilation. VCap also gives information related to the functional units A and C.

VCap calculates dead space (unit C) non-invasively using Bohr's formula[10] (Eq. 6):

$$\frac{VD_{Bohr}}{VT} = \frac{PACO_2 - P\bar{E}CO_2}{PACO_2} \qquad \text{(Eq. 6)}$$

where the alveolar partial pressure of $CO_2$ ($PACO_2$) may be determined as the $CO_2$ value found at the midpoint of the alveolar slope (Phase III) of the capnogram within the alveolar tidal volume[7,11]. The mixed partial pressure of $CO_2$ of an entire breath ($P\bar{\bar{E}}CO_2$) may also be non-invasively calculated from VCap using the following equation[18]:

$$P\bar{\bar{E}}CO_2 = \frac{VTCO_{2,br}}{VT} * (BP - PH_2O) \qquad \text{(Eq. 7)}$$

where $VTCO_{2,br}$ is the area under the curve of the VCap, BP is the barometric pressure, $PH_2O$ is the water vapour pressure and VT is the tidal volume.

Enghoff's formula (Eq. 8) was originally described to calculate a "surrogate of dead space" replacing $PACO_2$ by the arterial $PCO_2$ ($PaCO_2$), in Bohr's original formula[12] as:

$$\frac{VD_{B-E}}{VT} = \frac{PaCO_2 - P\overline{E}CO_2}{PaCO_2} \qquad (Eq.\ 8)$$

This formula was used in the past because $PACO_2$ was not available at the bedside. However, Enghoff's formula overestimates dead space because it replaces the alveolar $PCO_2$ by the arterial $PCO_2$ and thus includes all types of V/Q abnormalities beyond dead space in the calculation[18,19].

VCap is related to shunt (unit A) because it is known that the difference between Bohr's formula (Eq. 6) and Enghoff's formula (Eq. 8) is caused by a fictitious "alveolar dead space" caused by shunt. This *shunt dead space effect* has been well described in respiratory physiology[13,14].

Fig. 3C illustrates the VCap and its relation to dead space and the shunt effect, as described above. The gradient between mean alveolar ($PACO_2$) to mixed expired ($P\overline{E}CO_2$) partial pressure of $CO_2$ represents the true dead space or Bohr's dead space ($VD_{Bohr}/VT$), calculated using Bohr's formula (Eq. 6). The gradient between arterial partial pressure of $CO_2$ ($PaCO_2$) and $PECO_2$ represents a global index of the inefficiencies of gas exchange ($VD_{B-E}/VT$) that includes all types of V/Q abnormalities, calculated using the Bohr-Enghoff formula (Eq. 8). The differences between these formulas represent the shunt effect on dead space (hatched area). $VTCO_{2,br}$ is the area under the curve of VCap.

Considering the VCap and its relationship to dead space and the shunt effect described above, the present invention presents a novel approach in respiratory medicine wherein shunt is calculated using the kinetics of $CO_2$ instead of the one of $O_2$. Previous publications[15-18] analyzed the correction of the shunt effect on dead space but did not investigate the possibility to measure shunt using $CO_2$.

According to the inventive concept, one of two novel formulas may be used to calculate the shunt of a subject using parameters minimally-invasively derived from $CO_2$ measurements on expiration gas exhaled by said subject, preferably by means of volumetric capnography, together with values indicative of arterial $CO_2$ and cardiac output or EPP of the subject. The new formulas add two important components of the $CO_2$ kinetics that are related to shunt, namely the $CO_2$ transport by blood and its elimination by ventilation. The formulas are algebraically derived from equation 2A (Fick's equation for $Q_T$), equation 2B (Fick's equation for EPP), and equation 3 (Berggren's equation replacing $O_2$ by $CO_2$) as will be described in the following.

An important aspect of the present invention is the introduction of cardiac output ($Q_T$) or effective pulmonary perfusion (EPP) in the $CO_2$-based Berggren equation (Eq. 3) to eliminate the denominator ($CvCO_2 - CcCO_2$) and so the need for invasive measurements of venous blood content. Rearranging Fick's equations for EPP (Eq. 2B), the denominator in Berggren's equation (Eq. 3) can be expressed as:

$$\left(CvCO_2 - CcCO_2\right) = \frac{VCO_2}{EPP} \qquad (Eq.\ 9)$$

Combining equation 9 with the $CO_2$-based Berggren equation (Eq. 3) yields:

$$shunt\ (\%) = \frac{EPP * \left(CaCO_2 - CcCO_2\right)}{VCO_2} \qquad (Eq.10)$$

Another important aspect of the invention is the estimation of capillary $CO_2$ content from alveolar partial pressure, concentration or volume of $CO_2$ in order to replace the term $CcCO_2$ in the numerator of the $CO_2$-based Berggren equation (Eq. 3) with quantities that are either known or directly derivable from the $CO_2$ measurements on the expiration gas exhaled by the subject.

This is achieved according to a preferred embodiment of the invention by utilizing the fact that a value of alveolar partial pressure of $CO_2$ ($PACO_2$) substantially corresponding to the capillary partial pressure of $CO_2$ ($PcCO_2$) of the subject can be determined as a $CO_2$ value found at or near the midpoint of the alveolar slope of a volumetric capnogram directly obtained through said $CO_2$ measurements, and the fact that the capillary $CO_2$ content ($CcCO_2$) of the subject can be estimated from capillary partial pressure of $CO_2$ ($PcCO_2$) by using the following relationship:

$$CxCO_2 = S * PxCO_2 + B \qquad (Eq.\ 11)$$

where 5 is the $CO_2$ solubility, $PxCO_2$ is the partial pressure of $CO_2$ and $CxCO_2$ is the content of $CO_2$ in blood and B is the intercept of the straight line relating $CO_2$ partial pressure ($PxCO_2$) and content ($CxCO_2$) over a physiological range to be considered. This equation assumes that the $CO_2$ content is linearly related to the partial pressure of $CO_2$, something

that is true over the physiological range to be considered[15].

Considering equation 11 and the fact that the capillary partial pressure of $CO_2$ ($PcCO_2$) can be replaced by the $PACO_2$ value obtained from the volumetric capnogram as described above, the term $CcCO_2$ can be expressed as:

$$CcCO_2 = S * PcCO_2 + B = S * PACO_2 + B \qquad \text{(Eq. 12)}$$

where 5 is the $CO_2$ solubility in blood and B is the constant relating $PcCO_2$ to $CcCO_2$ over the physiological range to be considered.

Again considering equation 11, the arterial $CO_2$ content ($CaCO_2$) of the subject relates to the arterial partial pressure of $CO_2$ as:

$$CaCO_2 = S * PaCO_2 + b \qquad \text{(Eq. 13)}$$

where 5 is the $CO_2$ solubility in blood and b is a constant representing the intercept of the straight line relating $PaCO_2$ to $CaCO_2$ over the physiological range to be considered.

Now starting from equation 10 and combining this equation with equations 12 and 13, and assuming that the constants b and B for arterial and capillary blood are equal, shunt can be calculated as:

shunt (%) =

$$\frac{EPP\,(CaCO_2\text{-}CcCO_2)}{VCO_2} = \frac{EPP((S*PaCO_2+b)\text{-}(S*PACO_2+B))}{VCO_2} = \frac{S * EPP\,(PaCO_2\text{-}PACO_2)}{VCO_2} \qquad \text{(Eq. 5)}$$

Thus, by studying the arterial to capillary $CO_2$ content difference ($C(a\text{-}c)CO_2$), and taking the steps of: 1) replacing the arterial $CO_2$ content ($CaCO_2$) with a known value of $CO_2$ solubility in blood 5, a value of arterial partial pressure of $CO_2$ ($PaCO_2$), and a constant b representing the intercept of the straight line relating $PaCO_2$ to $CaCO_2$ over the physiological range to be considered; 2) replacing the capillary $CO_2$ content ($CcCO_2$) with a known value of $CO_2$ solubility in blood 5, a value of alveolar partial pressure of $CO_2$ ($PACO_2$) representing a value of capillary partial pressure $CO_2$ ($PcCO_2$) and directly derivable from the $CO_2$ measurements of expiration gas, and a constant B representing the intercept of the straight line relating $PaCO_2$ to $CaCO_2$ over the physiological range to be considered; and 3) assuming that the constants $b$ and $B$ are equal over the physiological range to be considered, the arterial to capillary $CO_2$ content difference can be replaced by an arterial to alveolar $CO_2$ partial pressure difference multiplied by a value 5 of $CO_2$ solubility in blood. Or, from another point of view, the arterial partial pressure of $CO_2$ and the alveolar partial pressure of $CO_2$, the latter being directly derivable from the $CO_2$ measurements on expiration gas, can be used to estimate the arterial to capillary difference of $CO_2$ content using the $CO_2$ solubility in blood 5, thus eliminating the need for determining not only the capillary $CO_2$ content ($CcCO_2$) but also the arterial $CO_2$ content ($CaCO_2$) of the subject.

The $CO_2$ elimination ($VCO_2$) of the subject may be calculated based on the $CO_2$ measurements on the expiration gas exhaled by the patient, and preferably based on volumetric capnography as:

$$VCO_2 = VTCO_{2,br} * RR \qquad \text{(Eq. 14)}$$

where $VCO_2$ is the elimination $CO_2$ per minute derived non-invasively from the area under the curve of the VCap ($VTCO_{2,br}$) multiplied by the respiratory rate (RR) of the subject.

Furthermore, considering that the shunt value calculated through equation 5 is the fraction of the cardiac output not participating in blood gas exchange, i.e. that:

$$shunt\,(\%) = \frac{Q_S}{Q_T} \qquad \text{(Eq. 15)}$$

where Qs is the shunt flow of blood not participating in blood gas exchange, and the fact that the cardiac output ($Q_T$) of the subject is the sum of the shunt flow ($Q_S$) and the effective pulmonary perfusion (EPP), i.e. that:

$$Q_T = EPP + Q_S \qquad \text{(Eq. 16)}$$

Then, by replacing EPP with $(Q_T - Q_S)$ in equation 5 and solving the equation for shunt, i.e. $Q_S/Q_T$, the following expression can be obtained:

$$\text{shunt (\%)} = \frac{S\,(PaCO_2\text{-}PACO_2)}{S\,(PaCO_2\text{-}PACO_2) + \dfrac{VCO_2}{Q_T}} \qquad \text{(Eq.4)}$$

The proposed principle for calculating shunt does not require any arterial or capillary $CO_2$ content to be calculated in absolute terms. Instead, by looking only at the difference in the arterial and capillary $CO_2$ content ($C(a\text{-}c)CO_2$), replacing the difference in arterial and capillary contents of $CO_2$ with the difference in arterial and capillary partial pressures of $CO_2$ ($P(a\text{-}c)CO_2$), and by replacing the capillary partial pressure of $CO_2$ with a corresponding alveolar partial pressure of $CO_2$ that can be determined from non-invasive $CO_2$ measurements on expiration gas, the present invention allows shunt to be calculated based on $CO_2$ measurements on expiration gas, a value of EPP or $Q_T$, a value of arterial $CO_2$ content and a value of $CO_2$ solubility in blood.

An advantage of these formulas is that shunt can be estimated without having to use a PAC by for example using volumetric capnography, a method for obtaining $Q_T$ or EPP, and an arterial blood sample to determine $PaCO_2$. The formula avoids complications and hospital costs related to the use of PAC. Furthermore, it is less dependent on the effects of differences in $FiO_2$ during shunt determination than other known formulas for shunt estimation. Yet further, the proposed $CO_2$ based approach for calculating shunt has several advantages compared to known $O_2$ based approaches for calculating shunt, as previously described in the summary of the invention.

Currently there are several described methods for non-invasive estimation of $Q_T$. These methods could enhance the usefulness of the above formula without increasing the need of invasive devices in clinical practice. Some of these methods are based on the application of the Fick principle to expired $CO_2$ analysis. However, as $CO_2$ delivery from blood to the alveolar gas requires the presence of effective capillary-alveolar exchange, these methods are closer to effective pulmonary perfusion (EPP) than to total cardiac output. Since these methods together with the proposed method for calculating shunt are based on capnography analysis and $PaCO_2$ of the subject, it could be advantageous to use equation 5 instead of equation 4 in the calculation of shunt.

One method that is particularly suitable for determination of $Q_T$ or EPP is a non-invasive capnodynamic method described in EP 2 641 536, which method is based on a capnodynamic equation describing how the fraction of alveolar carbon dioxide (FACO2) varies between different respiratory cycles. This method is advantageous not only because it is non-invasive but also because $Q_T$ or EPP can be determined only based on $CO_2$ measurements and calculations of $CO_2$ related parameters. Other methods that may also be employed for non-invasive determination of $Q_T$ or EPP within the scope of this invention are described in the background of EP 2 641 536, in US 6 042 550, and in Peyton et al[8].

As previously mentioned, the proposed method for shunt calculation may be completely non-invasive if a value of $PaCO_2$ is derived without an arterial blood sample. Therefore known surrogates of $PaCO_2$, such as partial pressure of end-tidal $CO_2$ ($PetCO_2$), may be used instead of $PaCO_2$ in the shunt calculation although use of such $PaCO_2$ surrogates reduces the accuracy in the shunt calculation. In the future, if a method for estimating $PaCO_2$ non-invasively becomes available, or if transcutaneous $PCO_2$ measurements become more reliable, clinicians will potentially have both a fully non-invasive and reliable method for estimating shunt at the bedside.

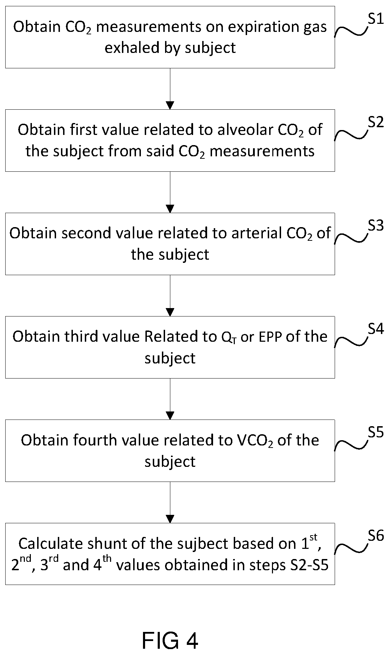

Fig. 4 illustrates a method for estimating shunt of a subject according to the principles of the invention. The method will be described with simultaneous reference to the previously described drawings.

In a first step S1, measurement values from $CO_2$ measurements on expiration gas exhaled by the subject are obtained by the apparatus 1A, 1B. These values typically include values of the flow or the volume of expiration gas exhaled by the subject and the partial pressure, concentration or volume of $CO_2$ in the expiration gas, measured by the capnograph 13 and transmitted to the apparatus 1A, 1B where they are received and used by the processing unit 21 in the calculation of shunt.

In a second step S2, a first value relating to alveolar $CO_2$ of the subject is obtained by the processing unit 21. Typically, said first value relating to alveolar $CO_2$ is obtained by the processing unit 21 by determining, based on the $CO_2$ measurements obtained in step S1, a value of alveolar $CO_2$ partial pressure, concentration or volume substantially corresponding to a capillary $CO_2$ partial pressure, concentration or volume of the subject. In a preferred embodiment, the alveolar $CO_2$ related value is a value of alveolar partial pressure of $CO_2$ ($PACO_2$) determined by the processing unit 21 based on the capnographic data received from the capnograph 13. Preferably, the $PACO_2$ value is determined based on a $CO_2$ value found at or near the midpoint of an alveolar slope (phase III) of a volumetric capnogram 23 derivable by the processing unit 21 based on the capnographic data.

In a third step S3, a second value related to arterial $CO_2$ of the subject is obtained by the processing unit 21, typically in form of a value of arterial $CO_2$ partial pressure ($PaCO_2$), concentration or volume. Preferably, the arterial $CO_2$ value is determined through analysis of blood gases in an arterial blood sample and input to the apparatus 1A, 1B, e.g. in form of a $PaCO_2$ value, via the user interface 31, whereupon it is received by the processing unit 21 and used in the determination of shunt.

In a fourth step S4, a third value related to the cardiac output ($Q_T$) or effective pulmonary perfusion (EPP) of the subject is obtained. As discussed above, the $Q_T$ or EPP-related value may be determined by the processing unit 21 based on the $CO_2$ measurements obtained in step S1, e.g. based on the capnographic data received from the capnograph 13, or be received by the processing unit 21 through manual input of a $Q_T$ or EPP-related value via the user interface 31 of the apparatus 1A, 1B.

In a fifth step S5, a fourth value related to $CO_2$ elimination ($VCO_2$) in the subject is obtained. Preferably, this value is determined by the processing unit 21 based on the $CO_2$ measurements obtained in step S1, e.g. based on the capnographic data received from the capnograph 13.

In a sixth and last step S6, the shunt of the subject is calculated by the processing unit 21 based on the first value related to alveolar $CO_2$ of the subject obtained in step S2, the second value related to arterial $CO_2$ of the subject obtained in step S3, the third value related to $Q_T$ or EPP of the subject obtained in step S4, and the fourth value related to $VCO_2$ of the subject obtained in step S5. As discussed above, the calculation of shunt preferably involves the step of combining a modified version of Berggren's equation where $O_2$ is replaced by $CO_2$ (Eq. 3) with Fick's equation for $Q_T$ or EPP (Eq. 2A and 2B, respectively) in order to eliminate the need for determining a venous $CO_2$ content of the subject. Furthermore, the calculation of shunt preferably involves the step of using the first and second values obtained in steps S2 and S3 to eliminate the need for determining a capillary $CO_2$ content of the subject. This may be achieved by using said first and second values to estimate a difference in arterial to capillary $CO_2$ content ($C(a-c)CO_2$), which has the further advantage of eliminating the need for determining an arterial $CO_2$ content of the subject. In a preferred embodiment, the calculation of shunt involves the steps of replacing, the arterial to capillary $CO_2$ content difference ($C(a-c)CO_2$) in the numerator of said $CO_2$-based Berggren equation (Eq. 3) with a difference in arterial to capillary partial pressure of $CO_2$ ($P(a-c)CO_2$), and using the first value related to alveolar $CO_2$ obtained in step S1 as a measure of capillary partial pressure of $CO_2$ of the subject. The replacement of the difference in arterial to capillary $CO_2$ content ($C(a-c)CO_2$) with the difference in arterial to capillary partial pressure of $CO_2$ ($P(a-c)CO_2$) further requires the $CO_2$ solubility in blood to be introduced and used in the calculation of shunt. Thus, in a preferred embodiment of the invention, the shunt of the subject is calculated based on the first to fourth values obtained in steps S2 to S5, and a value of $CO_2$ solubility in blood.

Preferably, the above described method is performed repetitively, e.g. on a breath-by-breath basis, in order to continuously monitor the shunt of the subject 3. That the method is repeated on a breath-by-breath basis here means that step S6 and at least one of the steps S2-S5 are repeated on a breath-by-breath basis in order to calculate an updated shunt value for each breath of the subject.

It should be noted that although the invention has herein been described as a method using a value of cardiac output ($Q_T$) or effective pulmonary perfusion (EPP) and a value of $CO_2$ elimination ($VCO_2$) of the subject in the calculation of shunt, it should be appreciated that the above described principles of using the alveolar $CO_2$ partial pressure, concentration or volume of the subject to eliminate the need for determining the capillary $CO_2$ content of the subject may be advantageously used also in existing or future methods for calculating shunt without using values of $Q_T$, EPP or $VCO_2$. Thus it should be appreciated that according to one aspect of the invention, there is provided a method for estimating shunt of a subject, comprising the steps of:

- obtaining, from $CO_2$ measurements on expiration gas exhaled by said subject, a first value related to alveolar $CO_2$ of said subject;
- obtaining a second value related to arterial $CO_2$ of said subject, and
- calculating the shunt of the subject based on said first and second values,

wherein said first value related to alveolar $CO_2$ is determined as a value of alveolar partial pressure, concentration or volume of $CO_2$ substantially corresponding to a capillary $CO_2$ partial pressure ($PcCO_2$), concentration or volume of the subject, and wherein said first value related to alveolar $CO_2$ is used in the calculation of shunt in a way that eliminates the need for determining a capillary $CO_2$ content ($CcCO_2$) of the subject.

As discussed above, said first value related to alveolar $CO_2$ may be a value of alveolar $CO_2$ partial pressure [$PACO_2$], concentration or volume, and said second value related to arterial $CO_2$ may be a value of arterial $CO_2$ partial pressure [$PaCO_2$], concentration or volume, which first and second values may be used together with a known value of $CO_2$ solubility in blood to estimate a difference in arterial to capillary $CO_2$ content ($C(a-c)CO_2$), thereby eliminating the need for determining the $CcCO_2$ of the subject.

Although the present invention has been described in connection with the specified embodiments, it should not be construed as being in any way limited to the presented examples. The scope of the present invention is to be interpreted

## EP 3 082 594 B1

in the light of the accompanying claim set. In the context of the claims, and other parts of the description, the terms "comprising" or "comprises" do not exclude other possible elements or steps.

Also, the mentioning of references such as "a" or "an" etc. should not be construed as excluding a plurality. The use of reference signs in the claims with respect to elements indicated in the figures shall also not be construed as limiting the scope of the invention. Furthermore, individual features mentioned in different claims, may possibly be advantageously combined, and the mentioning of these features in different claims does not exclude that a combination of features is not possible and advantageous.

Abbreviations, acronyms and definitions

| | |
|---|---|
| alveolar dead space | Ventilated alveoli not perfused by blood, i.e. alveoli for which the V/Q ratio approaches infinity |
| anatomic shunt | The fraction of blood bypassing the alveoli of the lungs through anatomic channels |
| BP | Barometric pressure |
| $CaCO_2$ | Arterial content of $CO_2$ |
| $CaO_2$ | Arterial content of $O_2$ |
| cardiac output | The volume of blood leaving the left (or right) ventricle each minute |
| $CcCO_2$ | Capillary content of $CO_2$ |
| $CcO_2$ | Capillary content of $O_2$ |
| $CvCO_2$ | Venous content of $CO_2$ |
| $CvO_2$ | Venous content of $O_2$ |
| Dead space | The portion of ventilation not participating in gas exchange |
| EPBF | Effective pulmonary blood flow |
| EPP | Effective pulmonary perfusion |
| $FiO_2$ | Inspired oxygen fraction |
| PAC | Pulmonary artery catheter |
| $PaCO_2$ | Arterial partial pressure of $CO_2$ |
| $PaO_2$ | Arterial partial pressure of $O_2$ |
| $PACO_2$ | Alveolar partial pressure of $CO_2$ |
| $P\bar{E}CO_2$ | Mixed expired partial pressure of $CO_2$ of an entire breath |
| $PH_2O$ | Water vapour pressure |
| pulmonary shunt | The fraction of blood perfusing the alveoli of the lungs not participating in gas exchange due to insufficient ventilation, i.e. shunt caused by zero or low V/Q ratio; corresponding to *venous admixture* |
| pure shunt | The fraction of pulmonary shunt caused by a V/Q ratio of zero |
| $Q_T$ | Cardiac output |
| RR | Respiratory rate |
| shunt | The (total) fraction of blood not involved in gas exchange; the sum of *anatomic shunt* and *pulmonary shunt* |
| VCap | Volumetric capnography |
| $VCO_2$ | Eliminated volume of $CO_2$ per minute ($CO_2$ elimination); sometimes referred to as $CO_2$ production since it corresponds to the litres of $CO_2$ produced by the tissues per minute |
| $VD_{Bohr}/VT$ | True dead space or Bohr's dead space; calculated as the gradient between mean alveolar ($PACO_2$) and mixed expired partial pressure of $CO_2$ ($P\bar{E}CO_2$) over $PACO_2$ |
| $VD_{B-E}/VT$ | Bohr-Enghoff's surrogate of dead space; calculated as the gradient between arterial partial pressure of $CO_2$ ($PaCO_2$) and mixed expired partial pressure of $CO_2$ ($P\bar{E}CO_2$) over $PaCO_2$ |
| venous admixture | *See pulmonary shunt* |
| V/Q heterogeneity | simultaneous presence of areas of the lung with low (non-zero) and high V/Q ratios |
| V/Q ratio | ventilation-perfusion ratio; the ratio of the amount of air reaching the alveoli to the amount of blood reaching these alveoli |
| VT | tidal volume |
| $VTCO_{2,br}$ | the area under the curve of the capnogram or the amount of $CO_2$ eliminated per breath, or minute $CO_2$ elimination divided by respiratory rate |

References

1. Berggren SM. The oxygen deficit of arterial blood cause by non-ventilating parts of the lung. Acta Physiol Scand 1942; 4: 1-92.

2. Benatar SR, Hewllet AM, Nunn JF. The use of iso-shunt lines for control of oxygen therapy. Br J Anaesth 1973; 45: 711-718.

3. Chiang ST. A nomogram for venous shunt (QS/QT) calculation. Thorax 1968; 23: 563-565.

4. Sapsford DJ, Jones JG. The PIO2 vs SpO2 diagram: a non-invasive measure of pulmonary oxygen exchange. Eur J Anaesth 1995; 12: 375-368.

5. Smith HL, Jones JG. Non-invasive assessment of shunt and ventilation/perfusion ratio in neonates with pulmonary failure. Arch Dis Child Fetal Neonatal Ed 2001; 85: F127-132.

6. Paulev PE, Siggaard-Andersen O. Clinical application of the PO2-PCO2 diagram. Acta Anaesthesiol Scand 2004; 48: 1105-1114.

7. Tusman G, Suarez-Sipmann F, Borges JB, Hedenstierna G, Bohm SH. Validation of Bohr dead space measured by volumetric capnography. Intensive Care Med 2011; 37: 870-874.

8. Peyton PJ, Poustie SJ, Robinson GJB, Penny DJ, Thompson B. Non-invasive measurement of intrapulmonary shunt during inert gas rebreathing. Physiological Measurement 2005; 26: 309-316.

9. Riley RL, Cournand A, 1949. Ideal alveolar air and the analysis of ventilation-perfusion relationships in the lungs. J Applied Physiol 1, 825-847.

10. Bohr C. Über die Lungenatmung. Skand Arch Physiol 1981; 2: 236-238.

11. Tusman G, Suarez-Sipmann F, Bohm SH. Rationale of dead space measured by volumetric capnography. Anesth Analg 2012; 114: 866-874.

12. Enghoff H. Volumen inefficax. Bemerkungen zur Frage des schädlichen Raumes. Uppsala Läkareforen Forhandl 1938; 44: 191-218.

13. Tang Y, Turner MJ, Baker AB, 2005. Effects of alveolar dead space, shunt and V/Q distribution on respiratory dead space measurements. Br J Anaesth 99, 538-548.

14. Suarez-Sipmann F, Santos A, Bohm SH, Borges JB, Hedenstierna G, Tusman G. Corrections of Enghoff's dead space formula for shunt effects still overestimate Bohr's dead space. Respir Physiol Neurobiol 2013; 189: 99-105.

15. Mecikalski MB, Cutillo A, Renzetti AD, 1984. Effect of right-to-left shunting on alveolar dead space. Bull Eur Physiophatol Respir 20, 513-519.

16. Torda TA, Duncalf D, 1974. Physiologic deadspace-to-tidal volume ratio: a new working equation and nomograms. Anesthesiology 40, 593-595.

17. Kuwabara S, Duncalf D. Effect of anatomic shunt on physiological dead space o ratio - a new equation. Anesthesiology 1969; 31:575-577.

18. Niklason L, Eckerström J, Jonson B, 2008. The influence of venous admixture on alveolar dead space and carbon dioxide exchange in acute respiratory distress syndrome: computer modelling. Crit Care 12, R53.

**Claims**

1. A computer-implemented method for estimating shunt of a subject (3), comprising the steps of:

   - obtaining (S2), from carbon dioxide [$CO_2$] measurements on expiration gas exhaled by said subject, a first value related to alveolar $CO_2$ of said subject;
   - obtaining (S3) a second value related to arterial $CO_2$ of said subject;
   - obtaining (S4) a third value related to cardiac output [$Q_T$] or effective pulmonary perfusion [EPP] of said subject;
   - obtaining (S5) a fourth value related to $CO_2$ elimination [$VCO_2$] of said subject, and
   - calculating (S6) the shunt of the subject based on said first, second, third and fourth values, **characterised in that** the $CO_2$ measurements are obtained by means of volumetric capnography, and wherein said first value related to alveolar $CO_2$ is determined based on capnographic data obtained through said volumetric capnography.

2. The method according to claim 1, wherein said first value related to alveolar $CO_2$ is a value of alveolar $CO_2$ partial pressure [$PACO_2$], concentration or volume, and said second value related to arterial $CO_2$ is a value of arterial $CO_2$ partial pressure [$PaCO_2$], concentration or volume, wherein said first and second values are used in the calculation of shunt to eliminate the need for determining a capillary $CO_2$ content [$CcCO_2$] of the subject.

3. The method according to claim 2, wherein said first and second values are used to estimate a difference in arterial to capillary $CO_2$ content [$C(a-c)CO_2$] of the subject (3) using a known value of $CO_2$ solubility in blood [$S$].

4. The method according to any of the preceding claims, wherein said first value related to alveolar $CO_2$ is determined as a value of alveolar $CO_2$ partial pressure [$PACO_2$], concentration or volume substantially corresponding to a capillary $CO_2$ partial pressure [$PcCO_2$], concentration or volume of the subject.

5. The method according to any of the preceding claims, wherein said first value related to alveolar $CO_2$ is determined based on a $CO_2$ value found at or near a midpoint of an alveolar slope of a volumetric capnogram (23) derivable from said capnographic data.

6. The method according to any of the preceding claims, wherein the shunt of the subject is calculated only from $CO_2$ related parameters.

7. A computer program for estimating shunt of a subject (3), comprising program code which, when executed by a processing unit (21) of an apparatus (1A; 1B), causes the apparatus of claims 8 to 14 to perform the method according to any of the preceding claims.

8. An apparatus (1A; 1B) for estimating shunt of a subject (3), comprising a processing unit (21) configured to:

   - obtain, from carbon dioxide [$CO_2$] measurements on expiration gas exhaled by said subject, a first value related to alveolar $CO_2$ of said subject;
   - obtain a second value related to arterial $CO_2$ of said subject;
   - obtain a third value related to cardiac output [$Q_T$] or effective pulmonary perfusion [EPP] of said subject;
   - obtain a fourth value related to $CO_2$ elimination [$VCO_2$] of said subject, and
   - calculate the shunt of the subject based on said first, second, third and fourth values, **characterised in that** the $CO_2$ measurements are obtained by means of volumetric capnography, and wherein the processing unit (21) is configured to determine said first value related to alveolar $CO_2$ based on capnographic data obtained through said volumetric capnography.

9. The apparatus (1A; 1B) according to claim 8, wherein said first value related to alveolar $CO_2$ is a value of alveolar $CO_2$ partial pressure [$PACO_2$], concentration or volume, and said second value related to arterial $CO_2$ is a value of arterial $CO_2$ partial pressure [$PaCO_2$], concentration or volume, and wherein the processing unit (21) is configured to use said first and second values in the calculation of shunt to eliminate the need for determining a capillary $CO_2$ content [$CcCO_2$] of the subject.

10. The apparatus (1A; 1B) according to claim 9, wherein the processing unit (21) is configured to use said first and second values to estimate a difference in arterial to capillary $CO_2$ content [$C(a-c)CO_2$] of the subject (3) using a known value of $CO_2$ solubility in blood [$S$].

11. The apparatus (1A; 1B) according to any of the claims 8 to 10, wherein the processing unit (21) is configured to determine said first value related to alveolar $CO_2$ as a value of alveolar $CO_2$ partial pressure [$PACO_2$], concentration or volume substantially corresponding to a capillary $CO_2$ partial pressure [$PcCO_2$], concentration or volume of the subject.

12. The apparatus (1A; 1B) according to any of the claims 8 to 11, wherein the processing unit (21) is configured to determine said first value related to alveolar $CO_2$ based on a $CO_2$ value found at or near a midpoint of an alveolar slope of a volumetric capnogram (23) derivable from said capnographic data.

13. The apparatus (1A; 1B) according to any of the claims 8 to 12, wherein the processing unit (21) is configured to calculate the shunt of the subject only from $CO_2$ related parameters.

14. The apparatus (1A; 1B) according to any of the claims 8 to 13, wherein the processing unit (21) is configured to calculate the shunt of the subject using a $CO_2$-based version of Berggren's equation for calculating shunt [Eq. 3], in which the value related to venous $CO_2$ content [$CvCO_2$] is eliminated by combining said $CO_2$ based version of Berggren's equation [Eq. 3] with the Fick equation for cardiac output [Eq. 2A] or effective pulmonary perfusion [Eq. 2B], and in which the value related to capillary $CO_2$ content [$CcCO_2$] is eliminated by use of said first value indicative of alveolar $CO_2$ content.

15. A ventilator (1A) for providing ventilatory support to a subject (3), wherein the ventilator comprises an apparatus according to any of the claims 8 to 14.

**Patentansprüche**

1. Computerimplementiertes Verfahren zum Schätzen des Shunts eines Individuums (3), umfassend die folgenden Schritte:

   - Erhalten (S2), aus Kohlendioxid- [$CO_2$-] Messungen an von dem Individuum ausgeatmetem Exspirationsgas, eines ersten Wertes in Bezug auf den alveolären $CO_2$ des Individuums;
   - Erhalten (S3) eines zweiten Wertes in Bezug auf den arteriellen $CO_2$ des Individuums;
   - Erhalten (S4) eines dritten Wertes in Bezug auf die Herzleistung [$Q_T$] oder die effektive pulmonale Perfusion [EPP] des Individuums;
   - Erhalten (S5) eines vierten Wertes in Bezug auf die $CO_2$-Elimination [$VCO_2$] des Individuums;
   - Berechnen (S6) des Shunts des Individuums basierend auf dem ersten, zweiten, dritten und vierten Wert,

   **dadurch gekennzeichnet, dass** die $CO_2$-Messungen mittels volumetrischer Kapnographie erhalten werden, wobei der erste Wert in Bezug auf den alveolären $CO_2$ basierend auf durch die volumetrische Kapnographie erhaltene kapnographische Daten bestimmt wird.

2. Verfahren nach Anspruch 1, wobei der erste Wert in Bezug auf den alveolären $CO_2$ ein Wert von alveolärem $CO_2$-Partialdruck [$PACO_2$], Konzentration oder Volumen ist, und der zweite Wert in Bezug auf den arteriellen $CO_2$ ein Wert eines arteriellen $CO_2$-Partialdrucks [$PaCO_2$], Konzentration oder Volumen ist, wobei der erste und zweite Wert beim Berechnen des Shunts zum Schätzen des Bedarfs des Bestimmens eines kapillaren $CO_2$-Gehalts [$CcCO_2$] des Individuums verwendet werden.

3. Verfahren nach Anspruch 2, wobei der erste und zweite Wert zum Schätzen eines Unterschieds zwischen arteriellem und kapillarem $CO_2$-Gehalt [$C(a-c)CO_2$] des Individuums (3) unter Verwendung eines bekannten Wertes von $CO_2$-Löslichkeit in Blut [S] verwendet werden.

4. Verfahren nach einem der vorstehenden Ansprüche, wobei der erste Wert in Bezug auf den alveolären $CO_2$ als ein Wert von alveolärem $CO_2$-Partialdruck [$PACO_2$], Konzentration oder Volumen bestimmt wird, der im Wesentlichen einem kapillaren $CO_2$-Partialdruck [$PaCO_2$], Konzentration oder Volumen des Individuums entspricht.

5. Verfahren nach einem der vorstehenden Ansprüche, wobei der erste Wert in Bezug auf den alveolären $CO_2$ basierend auf einem $CO_2$-Wert bestimmt wird, der an oder nahe einem Mittelpunkt einer Alveolarneigung eines volumetrischen Kapnogramms (23) gefunden wird, das aus den kapnographischen Daten ableitbar ist.

6. Verfahren nach einem der vorstehenden Ansprüche, wobei der Shunt des Individuums nur aus Parametern in Bezug auf $CO_2$ berechnet wird.

7. Computerprogramm zum Schätzen des Shunts eines Individuums (3), das Programmcode umfasst, welcher beim Ausführen durch eine Verarbeitungseinheit (21) einer Vorrichtung (1A; 1B) die Vorrichtung nach den Ansprüchen 8 bis 14 dazu veranlasst, das Verfahren nach einem der vorhergehenden Ansprüche durchzuführen.

8. Vorrichtung (1A; 1B) zum Schätzen des Shunts eines Individuums (3), umfassend eine Verarbeitungseinheit (21), die konfiguriert ist zum:

   - Erhalten, aus Kohlendioxid- [$CO_2$-] Messungen an von dem Individuum ausgeatmetem Exspirationsgas, eines ersten Wertes in Bezug auf den alveolären $CO_2$ des Individuums;
   - Erhalten eines zweiten Wertes in Bezug auf den arteriellen $CO_2$ des Individuums;
   - Erhalten eines dritten Wertes in Bezug auf die Herzleistung [$Q_T$] oder die effektive pulmonale Perfusion [EPP] des Individuums;
   - Erhalten eines vierten Wertes in Bezug auf die $CO_2$-Elimination [$VCO_2$] des Individuums;
   - Berechnen des Shunts des Individuums basierend auf dem ersten, zweiten, dritten und vierten Wert, **dadurch gekennzeichnet, dass** die $CO_2$-Messungen mittels volumetrischer Kapnographie erhalten werden, und wobei die Verarbeitungseinheit (21) konfiguriert ist, um den ersten Wert in Bezug auf den alveolären $CO_2$ basierend auf durch die volumetrische Kapnographie erhaltenen kapnographischen Daten zu bestimmen.

9. Vorrichtung (1A; 1B) nach Anspruch 8, wobei der erste Wert in Bezug auf den alveolären $CO_2$ ein Wert von alveolärem $CO_2$-Partialdruck [$PACO_2$], Konzentration oder Volumen ist, und der zweite Wert in Bezug auf den arteriellen $CO_2$

ein Wert eines arteriellen $CO_2$-Partialdrucks [$PaCO_2$], Konzentration oder Volumen ist, und wobei die Verarbeitungseinheit (21) konfiguriert ist, um den ersten und zweiten Wert beim Berechnen des Shunts zum Schätzen des Bedarfs des Bestimmens eines kapillaren $CO_2$-Gehalts [$CcCO_2$] des Individuums zu verwenden.

10. Vorrichtung (1A; 1B) nach Anspruch 9, wobei die Verarbeitungseinheit (21) konfiguriert ist, um den ersten und zweiten Wert zum Schätzen eines Unterschieds zwischen arteriellem und kapillarem $CO_2$-Gehalt [$C(a-c)CO_2$] des Individuums (3) unter Verwendung eines bekannten Wertes von $CO_2$-Löslichkeit in Blut [$S$] zu verwenden.

11. Vorrichtung (1A; 1B) nach einem der Ansprüche 8 bis 10, wobei die Verarbeitungseinheit (21) konfiguriert ist, um den ersten Wert in Bezug auf den alveolären $CO_2$ als einen Wert von alveolärem $CO_2$-Partialdruck [$PACO_2$], Konzentration oder Volumen zu bestimmen, der im Wesentlichen einem kapillaren $CO_2$-Partialdruck [$PaCO_2$], Konzentration oder Volumen des Individuums entspricht.

12. Vorrichtung (1A; 1B) nach einem der Ansprüche 8 bis 11, wobei die Verarbeitungseinheit (21) konfiguriert ist, um den ersten Wert in Bezug auf den alveolären $CO_2$ basierend auf einem $CO_2$-Wert zu bestimmen, der an oder nahe einem Mittelpunkt einer Alveolarneigung eines volumetrischen Kapnogramms (23) gefunden wird, das aus den kapnographischen Daten ableitbar ist.

13. Vorrichtung (1A; 1B) nach einem der Ansprüche 8 bis 12, wobei die Verarbeitungseinheit (21) konfiguriert ist, um den Shunt des Individuums nur aus Parametern in Bezug auf $CO_2$ zu berechnen.

14. Vorrichtung (1A; 1B) nach einem der Ansprüche 8 bis 13, wobei die Verarbeitungseinheit (21) konfiguriert ist, um den Shunt des Individuums unter Verwendung einer $CO_2$-basierten Version der Berggren-Gleichung zum Berechnen des Shunts [Eq. 3] zu berechnen, wobei der Wert in Bezug auf venösen $CO_2$-Gehalt [$CvCO_2$] durch Kombinieren der $CO_2$-basierten Version der Berggren-Gleichung [Eq. 3] mit der Fick-Gleichung für Herzleistung [Eq. 2A] oder effektive pulmonale Perfusion [Eq. 2B] eliminiert wird, und wobei der Wert in Bezug auf kapillaren $CO_2$-Gehalt [$CcCO_2$] eliminiert wird durch Verwenden des ersten Wertes, der alveolären $CO_2$-Gehalt angibt.

15. Beatmungsgerät (1A) zum Bereitstellen von Beatmungsunterstützung an ein Individuum (3), wobei das Beatmungsgerät eine Vorrichtung nach einem der Ansprüche 8 bis 14 umfasst.

## Revendications

1. Procédé mis en œuvre par ordinateur pour estimer la dérivation d'un sujet (3), comprenant les étapes consistant à :

- obtenir (S2), à partir des mesures de dioxyde de carbone [$CO_2$] sur le gaz d'expiration expiré par ledit sujet, une première valeur liée au $CO_2$ alvéolaire dudit sujet ;
- obtenir (S3) une deuxième valeur liée au $CO_2$ artériel dudit sujet ;
- obtenir (S4) une troisième valeur liée au débit cardiaque [$Q_T$] ou à la perfusion pulmonaire efficace [EPP] dudit sujet ;
- obtenir (S5) une quatrième valeur liée à l'élimination de $CO_2$ [$VCO_2$] dudit sujet, et
- calculer (S6) la dérivation du sujet sur la base desdites première, deuxième, troisième et quatrième valeurs,

**caractérisé en ce que** les mesures de $CO_2$ sont obtenues au moyen d'une capnographie volumétrique, et dans lequel ladite première valeur liée au $CO_2$ alvéolaire est déterminée sur la base de données capnographiques obtenues par ladite capnographie volumétrique.

2. Procédé selon la revendication 1, dans lequel ladite première valeur liée au $CO_2$ alvéolaire est une valeur de la pression partielle du $CO_2$ alvéolaire [$PACO_2$], concentration ou volume, et ladite deuxième valeur liée au $CO_2$ artériel est une valeur de la pression partielle du $CO_2$ artériel [$PaCO_2$], concentration ou volume, lesdites première et deuxième valeurs étant utilisées dans le calcul de la dérivation pour éliminer le besoin de déterminer une teneur en $CO_2$ capillaire [$CcCO_2$] du sujet.

3. Procédé selon la revendication 2, dans lequel lesdites première et deuxième valeurs sont utilisées pour estimer une différence de teneur en $CO_2$ artériel à capillaire [$C(a-c)CO_2$] du sujet (3) en utilisant une valeur connue de solubilité du $CO_2$ dans le sang [$S$].

**4.** Procédé selon l'une quelconque des revendications précédentes, dans lequel ladite première valeur liée au $CO_2$ alvéolaire est déterminée comme une valeur de pression partielle de $CO_2$ alvéolaire [$PACO_2$], concentration ou volume correspondant essentiellement à une pression partielle de $CO_2$ capillaire [$PcCO_2$], concentration ou volume du sujet.

**5.** Procédé selon l'une quelconque des revendications précédentes, dans lequel ladite première valeur liée au $CO_2$ alvéolaire est déterminée sur la base d'une valeur de $CO_2$ trouvée au niveau ou près d'un point médian d'une pente alvéolaire d'un capnogramme volumétrique (23) pouvant être déduit desdites données capnographiques.

**6.** Procédé selon l'une quelconque des revendications précédentes, dans lequel la dérivation du sujet est calculée uniquement à partir de paramètres liés au $CO_2$.

**7.** Programme informatique pour estimer la dérivation d'un sujet (3), comprenant un code de programme qui, lorsqu'il est exécuté par une unité de traitement (21) d'un dispositif (1A 1B), amène le dispositif selon les revendications 8 à 14 pour mettre en œuvre le procédé selon l'une quelconque des revendications précédentes.

**8.** Dispositif (1A; 1B) pour estimer la dérivation d'un sujet (3), comprenant une unité de traitement (21) configurée pour :

- obtenir, à partir des mesures de dioxyde de carbone [$CO_2$] sur le gaz d'expiration expiré par ledit sujet, une première valeur liée au $CO_2$ alvéolaire dudit sujet ;
- obtenir une deuxième valeur liée au $CO_2$ artériel dudit sujet ;
- obtenir une troisième valeur liée au débit cardiaque [$Q_T$] ou à la perfusion pulmonaire efficace [EPP] dudit sujet ;
- obtenir une quatrième valeur liée à l'élimination de $CO_2$ [$VCO_2$] dudit sujet, et
- calculer la dérivation du sujet sur la base desdites première, deuxième, troisième et quatrième valeurs,

**caractérisé en ce que** les mesures de $CO_2$ sont obtenues au moyen d'une capnographie volumétrique, et dans lequel l'unité de traitement (21) est configurée pour déterminer ladite première valeur liée au $CO_2$ alvéolaire sur la base de données capnographiques obtenues par ladite capnographie volumétrique.

**9.** Dispositif (1A; 1B) selon la revendication 8, dans lequel ladite première valeur liée au $CO_2$ alvéolaire est une valeur de la pression partielle du $CO_2$ alvéolaire [$PACO_2$], concentration ou volume, et ladite deuxième valeur liée au $CO_2$ artériel est une valeur de la pression partielle du $CO_2$ artériel [$PaCO_2$], concentration ou volume, et dans lequel l'unité de traitement (21) est configurée pour utiliser lesdites première et deuxième valeurs dans le calcul de la dérivation pour éliminer le besoin de déterminer une teneur en $CO_2$ capillaire [$CcCO_2$] du sujet.

**10.** Dispositif (1A; 1B) selon la revendication 9, dans lequel l'unité de traitement (21) est configurée pour utiliser lesdites première et deuxième valeurs pour estimer une différence de teneur en $CO_2$ artériel à capillaire [$C(a-c)CO_2$] du sujet (3) en utilisant une valeur connue de solubilité du $CO_2$ dans le sang [S].

**11.** Dispositif (1A; 1B) selon l'une quelconque des revendications 8 à 10, dans lequel l'unité de traitement (21) est configurée pour déterminer ladite première valeur liée au $CO_2$ alvéolaire comme une valeur de pression partielle de $CO_2$ alvéolaire [$PACO_2$], concentration ou volume correspondant essentiellement à une pression partielle de $CO_2$ capillaire [$PcCO_2$], concentration ou volume du sujet.

**12.** Dispositif (1A; 1B) selon l'une quelconque des revendications 8 à 11, dans lequel l'unité de traitement (21) est configurée pour déterminer ladite première valeur liée au $CO_2$ alvéolaire sur la base d'une valeur de $CO_2$ trouvée au niveau ou près d'un point médian d'une pente alvéolaire d'un capnogramme volumétrique (23) pouvant être déduit desdites données capnographiques.

**13.** Dispositif (1A; 1B) selon l'une quelconque des revendications 8 à 12, dans lequel l'unité de traitement (21) est configurée pour calculer la dérivation du sujet uniquement à partir de paramètres liés au $CO_2$.

**14.** Dispositif (1A; 1B) selon l'une quelconque des revendications 8 à 13, dans lequel l'unité de traitement (21) est configurée pour calculer la dérivation du sujet en utilisant une version basée sur $CO_2$ de l'équation de Berggren pour calculer la dérivation [Eq. 3], la valeur liée à la teneur en $CO_2$ veineux [$CvCO_2$] étant éliminée en combinant ladite version basée sur le $CO_2$ de l'équation de Berggren [Eq. 3] avec l'équation de Fick pour le débit cardiaque [Eq. 2A] ou une perfusion pulmonaire efficace [Eq. 2B], et la valeur liée à la teneur en $CO_2$ capillaire [$CcCO_2$] étant éliminée en utilisant ladite première valeur indicative de la teneur en $CO_2$ alvéolaire.

**15.** Ventilateur (1A) pour fournir une assistance respiratoire à un sujet (3), dans lequel le ventilateur comprend un appareil selon l'une quelconque des revendications 8 à 14.

FIG 1A

FIG 1B

FIG 2

## FIG 3A

## FIG 3B

## FIG 3C

Obtain $CO_2$ measurements on expiration gas exhaled by subject — S1

Obtain first value related to alveolar $CO_2$ of the subject from said $CO_2$ measurements — S2

Obtain second value related to arterial $CO_2$ of the subject — S3

Obtain third value Related to $Q_T$ or EPP of the subject — S4

Obtain fourth value related to $VCO_2$ of the subject — S5

Calculate shunt of the sujbect based on $1^{st}$, $2^{nd}$, $3^{rd}$ and $4^{th}$ values obtained in steps S2-S5 — S6

## FIG 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2012069051 A
- US 6042550 A, Peyton
- EP 2641536 A

### Non-patent literature cited in the description

- **BERGGREN SM.** The oxygen deficit of arterial blood cause by non-ventilating parts of the lung. *Acta Physiol Scand,* 1942, vol. 4, 1-92
- **BENATAR SR ; HEWLLET AM ; NUNN JF.** The use of iso-shunt lines for control of oxygen therapy. *Br J Anaesth,* 1973, vol. 45, 711-718
- **CHIANG ST.** A nomogram for venous shunt (QS/QT) calculation. *Thorax,* 1968, vol. 23, 563-565
- **SAPSFORD DJ ; JONES JG.** The PIO2 vs SpO2 diagram: a non-invasive measure of pulmonary oxygen exchange. *Eur J Anaesth,* 1995, vol. 12, 375-368
- **SMITH HL ; JONES JG.** Non-invasive assessment of shunt and ventilation/perfusion ratio in neonates with pulmonary failure. *Arch Dis Child Fetal Neonatal Ed,* 2001, vol. 85, F127-132
- **PAULEV PE ; SIGGAARD-ANDERSEN O.** Clinical application of the PO2-PCO2 diagram. *Acta Anaesthesiol Scand,* 2004, vol. 48, 1105-1114
- **TUSMAN G ; SUAREZ-SIPMANN F ; BORGES JB ; HEDENSTIERNA G ; BOHM SH.** Validation of Bohr dead space measured by volumetric capnography. *Intensive Care Med,* 2011, vol. 37, 870-874
- **PEYTON PJ ; POUSTIE SJ ; ROBINSON GJB ; PENNY DJ ; THOMPSON B.** Non-invasive measurement of intrapulmonary shunt during inert gas rebreathing. *Physiological Measurement,* 2005, vol. 26, 309-316
- **RILEY RL ; COURNAND A.** Ideal alveolar air and the analysis of ventilation-perfusion relationships in the lungs. *J Applied Physiol,* 1949, vol. 1, 825-847
- **BOHR C.** Über die Lungenatmung. *Skand Arch Physiol,* 1981, vol. 2, 236-238
- **TUSMAN G ; SUAREZ-SIPMANN F ; BOHM SH.** Rationale of dead space measured by volumetric capnography. *Anesth Analg,* 2012, vol. 114, 866-874
- **ENGHOFF H.** Volumen inefficax. Bemerkungen zur Frage des schädlichen Raumes. *Uppsala Läkareforen Forhandl,* 1938, vol. 44, 191-218
- **TANG Y ; TURNER MJ ; BAKER AB.** Effects of alveolar dead space, shunt and V/Q distribution on respiratory dead space measurements. *Br J Anaesth,* 2005, vol. 99, 538-548
- **SUAREZ-SIPMANN F ; SANTOS A ; BOHM SH ; BORGES JB ; HEDENSTIERNA G ; TUSMAN G.** Corrections of Enghoff's dead space formula for shunt effects still overestimate Bohr's dead space. *Respir Physiol Neurobiol,* 2013, vol. 189, 99-105
- **MECIKALSKI MB ; CUTILLO A ; RENZETTI AD.** Effect of right-to-left shunting on alveolar dead space. *Bull Eur Physiophatol Respir,* 1984, vol. 20, 513-519
- **TORDA TA ; DUNCALF D.** Physiologic dead-space-to-tidal volume ratio: a new working equation and nomograms. *Anesthesiology,* 1974, vol. 40, 593-595
- **KUWABARA S ; DUNCALF D.** Effect of anatomic shunt on physiological dead space o ratio - a new equation. *Anesthesiology,* 1969, vol. 31, 575-577
- **NIKLASON L ; ECKERSTRÖM J ; JONSON B.** The influence of venous admixture on alveolar dead space and carbon dioxide exchange in acute respiratory distress syndrome: computer modelling. *Crit Care,* 2008, vol. 12, R53